# EUROPEAN PATENT APPLICATION

(11) **EP 4 372 104 A1**
(43) Date of publication of application: **22.05.2024**
(21) Application number: 22842236.6
(22) Date of filing: 29.03.2022
(51) Int. Cl.: C12Q 1/689, C12Q 1/6834

(54) **COMPOSITION FOR DETECTING MICROBIAL ON-SITE CONTAMINATION AND USE THEREOF**

(30) Priority: 13.07.2021 KR 20210091587
(71) Applicant: Bionano Health Guard Research Center, Daejeon 34141 (KR)
(72) Inventor: SHIN, Yong Beom, Daejeon 34141 (KR); KIM, Sun Jung, Daejeon 34141 (KR); CHO, Hyun Min, Daejeon 34141 (KR)
(74) Representative: Cabinet Becker et Associés
(86) International application number: PCT/KR2022/004428
(87) International publication number: WO 2023/286973

(57) **Abstract**

The present invention relates to a composition for detecting on-site microbial contamination and uses thereof. The composition for detecting on-site microbial contamination, according to the present invention, is capable of detecting the nucleic acid decomposition ability of a comprehensive nuclease, and detecting a living microorganism in a sample very quickly and accurately, and a detection method thereby is very simple. Accordingly, the composition of the present invention is expected to be able to easily and conveniently measure the degree of contamination of microorganisms in an environment.

## Description

### [Technical Field]

The present disclosure relates to a composition for detecting microbial on-site contamination and uses thereof.

### [Background Art]

Pathogens are widely distributed in the surrounding environment and exist in many places in daily life. The human body also has, on average, at least 150 types of bacteria both inside and outside the body, and many of these microorganisms are harmless to the human body, but some types thereof may cause various infectious diseases, including botulism, cholera, diarrhea, emesis, pneumonia, and typhoid. The pathogens may easily infect humans through contaminated soil, water environments, food materials, cooking environments, etc. Since the propagation rate of pathogens is very fast under normal conditions, even when a small number of pathogens are once invaded into the human body, the pathogens rapidly grow in the intestine very suitable for their growth environment to reach a level that may threaten human health. Accordingly, there is a need for a diagnostic technique capable of quickly and easily detecting the presence or absence of pathogens from a contaminated environment. Pathogenic microorganisms are generally detected and identified using a medium method, and this method has a disadvantage in that it takes a long time for proliferation and culture of microorganisms. In addition, PCR and an enzyme-linked immunosorbent assay (ELISA) using antigen antibody have been used, which require expensive equipment and skilled technology, thereby making it difficult to quickly determine the presence or absence of microorganisms in the on-site. Accordingly, there is a need for a technology that is able to more conveniently and quickly determine the presence or absence of microorganisms in the on-site.

In addition, in general, the presence of microorganisms is confirmed in the on-site through ATP measurement, but since ATP is an energy source involved in the metabolism of living organisms and is produced not only by microorganisms but also by other organic materials, it is difficult to accurately measure the infection of pathogens, and even if the cells die, the function of ATP is maintained, so that there is a high possibility that microorganisms that are killed or have very low viability may also be measured. In order to solve this problem, it is necessary to measure the amount and activity of enzymes always present in cells to apply to detection of microorganisms.

### [Disclosure]

### [Technical Problem]

Accordingly, the present inventors have made many efforts to develop a method for detecting living microorganisms very quickly and effectively without expensive equipment and skilled experimenters. As a result, the present inventors developed a solid substrate-immobilized probe having one end bound to a solid substrate and the other end bound to an enzyme capable of exhibiting a signal to measure the activity of a nuclease present in cells. In addition, the inventors found that the probe was able to accurately and efficiently detect the nucleic acid decomposition ability of a nuclease of an infectious microorganism, and then completed the present disclosure.

Accordingly, an object of the present disclosure is to provide a composition for detecting microbial contamination, including a preparation for detecting a nuclease.

Another object of the present disclosure is to provide a kit for detecting microbial contamination.

Yet another object of the present disclosure is to provide a method for detecting microbial contamination.

Yet another object of the present disclosure is to provide an oligonucleotide for measuring the activity of a nuclease.

### [Technical Solution]

The terms used herein are used for the purpose of description only, and should not be construed to be limited. The singular expression includes the plural expression unless the context clearly dictates otherwise. In the present disclosure, it should be understood that term "comprising" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present disclosure.

Hereinafter, the present disclosure will be described in more detail.

According to an aspect of the present disclosure, the present disclosure provides a composition for detecting on-site microbial contamination comprising a solid surface-immobilized oligonucleotide probe which is immobilized through its 5' or 3'-end on the surface of the solid substrate.

According to a preferred embodiment of the present disclosure, any solid substrate may be used as long as the object of the present disclosure is able to be achieved. For example, the solid substrate may be at least one selected from the group consisting of well-plate, slide glass, column, porous support, magnetic nanoparticle, gold, alloy, aluminum, metal oxide, ceramic, quartz, silicon, semiconductor, Si/SiO₂ wafer, germanium, gallium arsenide, carbon, carbon nanotube, polystyrene, polyethylene, polypropylene, polyacrylamide, sepharose, agarose, and colloid, but is not limited thereto.

In one embodiment of the present disclosure, the magnetic nanoparticle is used as the solid substrate, and thus, there is provided a composition for detecting on-site microbial contamination including magnetic nanoparticles to which oligonucleotide probes are linked.

According to a preferred embodiment of the present disclosure, the composition of the present disclosure may be used to detect living microorganisms.

That is, a feature of the present disclosure is to diagnose the contamination of microorganisms using a nuclease, which is an enzyme present in living microorganisms.

Since the function of ATP is maintained even when cells die, a microbial assay commonly used in the art also measures microorganisms with very low viability or dead to cause false positive results.

However, since the composition of the present disclosure is able to substantially detect the presence of live microorganisms rapidly and easily, it is possible to solve the problem of false positives as described above.

In the present disclosure, the microorganism means bacteria or fungi.

The types of bacteria that may be detected by the composition of the present disclosure are not limited. Specifically, the bacteria may be Gram-negative bacteria or Gram-positive bacteria.

Examples of the Gram-negative bacteria may include *Escherichia coli (E. coli), Helicobcater, Hemophilus, Neisseria, Cyano bacteria, Klebsiella, Acetobacter, Enterobacter, Chlamydia, Vibrio, Pseudomona, Salmonella, Thiobacter, Borrelia, Burkholderia, Serratia, and Treponema.*

Examples of the Gram-positive bacteria may include *Bacillus, Nocardia, Clostridium, Propionibacterium, Actinomyces, Enterococcus, Cornyebacterium, Listria, Lactobacillus, Gardnerella, Mycobacterium, Mycoplasma, Staphylococcus, Streptomyces,* and *Streptococcus.*

Fungi that may be detected by the composition of the present disclosure are not limited. For example, the fungi may be *Fusarium spp., Penicillium spp.* or *Rhizoctonia solani* as plant pathogenic fungi, and may include *Candida spp., Aspergillus spp., Cryptococcus neoformans* and *Trichophyton spp.,* as animal pathogenic fungi.

As used herein, the term "magnetic nanoparticle linked to the oligonucleotide probe" refers to a magnetic nanoparticle linked to an oligonucleotide that is recognized as a target and cleaved by a nucleolytic reaction of a microbial nuclease, and is used interchangeably with a "DNA-binding magnetic nanoparticle" in the present disclosure.

The DNA-binding magnetic nanoparticle is not particularly limited as long as the object of the present disclosure is achieved, but for example, the nanoparticle may be a metal selected from the group consisting of Au, Pt, Pd, Ag and Cu; a magnetic material selected from the group consisting of Co, Mn, Fe, Ni, Gd, Mo, MM'₂O₄, and MₓO_{y} (M and M' each independently represent Co, Fe, Ni, Mn, Zn, Gd, or Cr, and 0 < x ≤, 0 < y ≤ 5); or a magnetic alloy selected from the group consisting of CoCu, CoPt, FePt, CoSm, NiFe and NiFeCo, preferably Fe.

According to a preferred embodiment of the present disclosure, the oligonucleotide probe includes a label generating a detectable signal at a 5' or 3' end, and the oligonucleotide probe is cleaved by a nucleolytic reaction of the microbial nuclease to release the label from the oligonucleotide probe, resulting in generation of the detectable signal.

The label may be a single label or a dual-label.

The label is not particularly limited as long as the object of the present disclosure is achieved, but for example, the label may be at least one selected from the group consisting of a chemical label, an enzymatic label, a radioactive label, a fluorescent label, a luminescent label, a chemiluminescent label, and a metal label.

For example, chemical labels (e.g., biotin), enzymatic labels (e.g., alkaline phosphatase, β-galactosidase and β-glucosidase, luciferase, cytochrome P450 and horseradish peroxidase), radioactive labels (e.g., C14, 1125, P32 and S35), fluorescent labels, luminescent labels, chemiluminescent labels or metallic labels (e.g., gold).

The label of the present disclosure is preferably a label capable of generating a signal in real time manner, and in one embodiment of the present disclosure, biotin was used as a chemical label.

The biotin binds to at least one biotin-affinity protein selected from the group consisting of streptavidin, avidin, traptavidin, and neutravidin, and the biotin-affinity protein is labeled with an enzyme.

The enzyme is not particularly limited as long as the object of the present disclosure is achieved, but for example, the enzyme may be at least one selected from the group consisting of horseradish peroxidase (HRP), alkaline phosphatase (ALP), luciferase, glucokinase, invertase, glucose oxidase, β-D-galactosidase, malate dehydrogenase (MDH) and acetylcholinesterase, and preferably horseradish peroxidase (HRP).

In addition, the label on the probe of the present disclosure is located at its 5'-end or a position separated by 1-5 nucleotides from the 5'-end, preferably its 5'-end or a position separated by 1-4 nucleotides from the 5'-end, more preferably its 5'-end or a position separated by 1-3 nucleotides from the 5'-end, much more preferably its 5'-end or a position separated by 1-2 nucleotides from the 5'-end, and most preferably its 5'-end.

According to one embodiment of the present disclosure, the oligonucleotide is single-stranded or double-stranded.

The term "5'-end" region used herein while referring to the probe refers to a region or zone that includes a continuous sequence of any length from the 5'-end of the probe. Preferably, the 5'-end region of the probe consists of a sequence including 1-10 nucleotides from its 5'-end.

The term "3'-end" region used herein while referring to the probe refers to a region or zone that includes a continuous sequence of any length from the 3'-end of the probe. Preferably, the 3'-end region of the probe consists of a sequence including 1-10 nucleotides from its 3'-end.

In the present disclosure, the nucleic acid strand refers to oligonucleotide, nucleotide or polynucleotide, and fragments or portions thereof, and refers to DNA or RNA of genomic or synthetic origin, which may refer to a sense or antisense strand. In the present disclosure, the nucleic acid strand may preferably consist of DNA.

In addition, the probe of the present disclosure is preferably characterized by consisting of a double nucleic acid strand. The double nucleic acid strands of the present disclosure are mutually hybridized. As used herein, the term "hybridization" is used in reference to pairing of complementary nucleic acids. The hybridization and hybridization strength (i.e., the strength of association between nucleic acids) are influenced by factors such as the degree of complementarity between nucleic acids, the stringency of relevant conditions, and the Tm of a formed hybrid. The "hybridization" method includes annealing of one nucleic acid and the other complementary nucleic acid, i.e., a nucleic acid having a complementary nucleotide sequence. The ability of two nucleic acid polymers containing complementary sequences to recognize each other and anneal through base pairing interactions is a well-understood phenomenon. The early observation of the process of "hybridization" (Marmur and Lane, Proc. Natl. Acad. Sci. USA 46:453 (1960) and Doty et al., Proc. Natl. Acad. Sci. USA 46:461 (1960)) was advanced by subsequent research and became an essential tool of modern biology.

In the nucleic acid strand of the present disclosure, bases that are not generally found in natural nucleic acids may be included in the nucleic acid of the present disclosure, and include, for example, inosine and 7-deazaguanine. The complementarity needs not be perfect; and stable dimers may include mismatched base pairs or unpaired bases. Those skilled in the art of nucleic acid technology may experimentally determine stability of the dimmers by considering a number of variables, such as the length of the oligonucleotide, the base composition and sequence of the oligonucleotide, the ionic strength, and the incidence of mismatched base pairs.

The nucleic acid strands of the present disclosure may be generated in any manner, for example, chemical synthesis, DNA replication, reverse transcription, PCR, or a combination thereof.

In the present disclosure, the nuclease means a nucleic acid hydrolase, and means an enzyme that catalyzes a hydrolysis reaction of nucleic acids, nucleotides, nucleosides, and the like. The nuclease is not limited in its kind, and includes DNase that degrades DNA and RNase that degrades RNA. In addition, the nuclease is a concept including polynucleotidase, nucleotidase, and nucleosidase. In addition, the nuclease includes exonuclease that sequentially decomposes the 3' end or 5' end and endonuclease that cleaves the inside of a nucleic acid chain. In particular, the present disclosure includes a configuration capable of simultaneously measuring the exonuclease or endonuclease.

In addition, the solid surface-immobilized oligonucleotide probe of the present disclosure may further bind to a separate nanoparticle, and the separate nanoparticle may be a labeling material immobilized on its surface.

That is, the solid surface-immobilized oligonucleotide probe of the present disclosure may additionally bind a labeled nanoparticle in order to increase measured signal generation, and as the additional separate nanoparticle, for example, a gold nanoparticle obtained by binding poly-HRP to a gold nanoparticle may be introduced.

In addition, the oligonucleotide probe may consist of a nucleotide sequence selected from the group consisting of nucleotide sequences of SEQ ID NOs: 1 to 5, and according to one embodiment of the present disclosure, in addition to the single-stranded state, two or more of the single-stranded nucleotide sequences may be combined and annealed to be prepared and used in a double-stranded state.

According to one embodiment of the present disclosure, the nanoparticle linked with the oligonucleotide probe was designed by binding single-stranded and double-stranded DNA structures to one oligonucleotide probe and immobilizing the magnetic nanoparticle thereto so that the DNA-binding magnetic nanoparticle of the present disclosure is decomposed by various DNA exonucleases to generate a signal. More preferably, the oligonucleotide probe of the present disclosure biotinylates the 5'-end so that a label generating a detectable signal may be bound to the probe, and was bound with streptavidin labeled with an enzyme that is friendly to the biotin and generates a detectable signal. In addition, the oligonucleotide probe has an amino group (NH₂) at the 3'-end, and was immobilized on the surface of the magnetic nanoparticle, which is a solid substrate through the amino group.

The magnetic nanoparticle to which the oligonucleotide probe of the present disclosure is linked may obtain a detection effect as long as the nucleic acid probe is cleaved by a nuclease produced during bacterial lysis, regardless of a single strand, a double strand, and its length. Accordingly, Gram-negative bacteria or Gram-positive bacteria may be quickly and easily detected without false-positive signals by using the microbial contamination detection technology using the magnetic nanoparticle linked to the probe of the present disclosure.

In addition, according to another aspect of the present disclosure, the present disclosure provides a kit for detecting on-site microbial contamination, including the composition.

The kit of the present disclosure may be used to detect microorganisms.

The kit of the present disclosure provides a kit for storage or delivery of reaction ingredients required to perform microbial detection. The kit may include any and all ingredients required or preferred for detection, and the ingredients include reagents themselves, buffers, control reagents (e.g., tissue samples, positive and negative control targeting oligonucleotides, etc.), solid supports, labels, written and/or pictorial instructions and product information, inhibitors, labeling and/or detection reagents, and packaging environmental controls (e.g., ice, dehumidifiers, etc.), but are not limited thereto.

Since the kit of the present disclosure includes the above-described composition as a configuration, the description of duplicated contents will be omitted in order to avoid excessive complexity of the present specification.

According to yet another aspect of the present disclosure, the present disclosure provides a method for diagnosing on-site microbial contamination, including the following steps:
(a) dissolving a microorganism in a sample;
(b) treating the sample with the aforementioned composition; and
(c) detecting a signal released from the sample.

In the present disclosure, the term "sample" is used in the broadest sense. Meanwhile, the sample is meant to include specimens or cultures (e.g., microorganism cultures). On the other hand, the sample is meant to include both biological and environmental samples. In addition, the sample may include synthetic-origin samples.

The biological sample may also be sterilized liquid and solid food and feed products and dairy items, ingredients such as vegetables, meat and meat by-products, and waste. The biological sample may be obtained from all livestock of various classes, including ferals or wild animals, and these animals include ungulates, bear, fish, lagomorpha, and rodents, but is not limited thereto.

The environmental sample includes environmental materials such as a surface matter, soil, water, and industrial samples, in addition to food and dairy processing equipment, tools, equipment, utensils, disposable and non-disposable items. These examples should not be construed as limiting the types of samples applicable to the present disclosure.

The method of the present disclosure may include a step of confirming color development as a signal in the sample. The color development may be confirmed by generating a signal (color-developing) by a chromogenic substrate of the exposed enzyme while the cleaved probe is released, at the time when the probe labeled with the enzyme generating the signal is cleaved by the nuclease of the microorganism when a microorganism is present in the sample.

For example, when a substrate solution such as TMB is added, in a negative sample without microorganisms, the probe is not cleaved and does not react with the substrate, so that a colorimetric (chromogenic) reaction does not occur, and in a positive sample with microorganisms, the probe is cleaved to react with the substrate, so that a strong colorimetric reaction occurs to detect the microbial contamination.

Since the method of the present disclosure detects the signal using the aforementioned composition, the description of duplicated contents will be omitted in order to avoid excessive complexity of the present specification.

### [Advantageous Effects]

According to the present disclosure, the composition for detecting on-site microbial contamination is capable of detecting the nucleic acid decomposition ability of a comprehensive nuclease, and detecting a living microorganism in a sample very quickly and accurately, and a detection method thereby is very simple. Accordingly, the composition of the present disclosure is expected to be able to easily and conveniently measure the degree of contamination of microorganisms in an environment.

### [Description of Drawings]

FIG. 1 schematically illustrates a method for detecting microbial contamination in the field by DNA-binding magnetic nanoparticles according to the present disclosure.
FIG. 2 illustrates a result of confirming the reactivity of the DNA-binding magnetic nanoparticles of the present disclosure to endonuclease (DNase).
FIG. 3 illustrates a result of *E. coli* detection of DNA-binding magnetic nanoparticles using a chromogenic reaction.
FIG. 4 illustrates a result of *E. coli* detection according to an amount of DNA-binding magnetic nanoparticles using a chromogenic reaction.
FIG. 5 illustrates a result of *Acinetobacter baumannii* detection of DNA-binding magnetic nanoparticles using a chromogenic reaction.
FIG. 6 illustrates a result of *Staphylococcus aureus* detection of DNA-binding magnetic nanoparticles using a chromogenic reaction.
FIG. 7 illustrates a result of *E. coli* detection of DNA-binding magnetic nanoparticles bound with each enzyme using a luminescent reaction.

### [Best Mode for the Invention]

Hereinafter, the present disclosure will be described in more detail with reference to Examples. However, these Examples are only illustrative for the present disclosure, and the scope of the present disclosure is not limited to these Examples.

### Example 1. Fabrication of magnetic nanoparticle linked with oligonucleotide probe

The present disclosure was performed with the support of the National Research Foundation of Korea with funding from the government (Ministry of Science and ICT) [Project unique number: 1711131054 (Research business name: Global Frontier Support (R&D), Research project name: Securing bio contents for H-GUARD system and developing technology for early detection/diagnosis commercialization); and Project unique number: 1711137223 (Research business name: Nano connect, Research project name: Development of infectious pathogen detection system in indoor living environment)]

The present inventors designed magnetic nanoparticles linked with probes by binding single-stranded and double-stranded DNA structures to one oligonucleotide probe and immobilizing the magnetic nanoparticles thereto so that the nanoparticles were decomposed by various DNA exonucleases to generate a signal.

More specifically, the oligonucleotide probe of the present disclosure biotinylated a 5'-end so that a label generating a detectable signal may be bound to the probe, and was bound with streptavidin labeled with an enzyme that was friendly to the biotin and generated a detectable signal. In addition, the oligonucleotide probe had an amino group (NH₂) at the 3'-end, and was immobilized on the surface of magnetic nanoparticles, which was a solid substrate through the amino group.

Oligonucleotide sequences were as follows:
DNA1: 5'-biotin-ATC ACC CTC CCC GCA CCT AAA GGG TGC GGG GAG GGT-NH₂(C6)3' (SEQ ID NO: 1)
DNA2: 5'-ACC CTC CCC GCA CCC TAT TGG TGC GGG GAG GG-3' (SEQ ID NO: 2)
DNA3: 5'-biotin-ACC CTC CCC GCA CCC TAT TGG TGC GGG GAG GG-NH₂(C6)3' (SEQ ID NO: 3)
DNA4: 5'-ATC ACC CTC CCC GCA CCT AAA GGG TGC GGG GAG GGT-3' (SEQ ID NO: 4)
DNA5: 5'-biotin TCG GGA CAC TGA ACC CTA AAG GGG TTC AGT GTC CC-NH₂ (C6)3' (hairpin) (SEQ ID NO: 5)

### (Underlines indicated mismatched nucleotides)

Oligonucleotides having biotin bound to a 5'-end and an amino group bound to a 3'-end were supplied from GenoTech (Korea).

The oligonucleotides were dissolved in 100 µM of distilled water at a high concentration. 10 µl of the oligonucleotides were mixed in 100 µl of an annealing buffer (50 mM NaCl, 10 mM Tris-HCl pH 7.9, 10 mM MgCl₂, 100 µg mL⁻¹ bovine serum albumin (BSA)). This annealing buffer provided a buffering environment and salts were essential for hybridization of oligonucleotides. The mixed oligonucleotides were incubated at 95°C for 5 minutes and then gradually cooled at 45°C for 70 minutes. The annealed probes may be stored at 4°C in a stable double-stranded form.

5 mg of 400 nm magnetic nanoparticles containing a carboxyl group (magnetic (Fe) nanoparticles surface-modified with carboxylic acid at 400 nm) were added to 100 µM biotin-DNA sequence-NH₂ of the oligonucleotide probe and then reacted with 1-ethyl-3-[3-dimethylaminopropyl]carbodiimide hydrochloride (EDC) and N-hydroxysuccinimide (NHS) for 24 hours.

Thereafter, an unreacted product was removed through a magnet, and streptavidin-HRP was bound with Streptavidin-HRP, or Streptavidin-poly HRP, or a gold nanoparticle, and reacted at room temperature for 2 hours. The unreacted product was removed through the magnet and re-dispersed in 100 µl of PBS.

In addition, in order to apply to the following Examples, magnetic nanoparticle experimental groups to which the probes were linked (coupled) were fabricated by combining the DNAs 1 to 5 (SEQ ID NOs: 1 to 5) as follows:
No. 1; Magnetic nanoparticle alone as untreated control,
No. 2; Magnetic nanoparticle bound with DNA1 and then annealed with DNA2,
No. 3; Magnetic nanoparticle bound with DNA3 and then annealed with DNA4,
No. 4; Magnetic nanoparticle bound with DNA5 (having hairpin structure),
No. 5; Annealed with DNA1 and DNA2 and then bound with magnetic nanoparticle
No. 6; Annealed with DNA3 and DNA4 and then bound with magnetic nanoparticle

### Example 2. Reactivity of magnetic nanoparticle linked with oligonucleotide probe with nuclease

The present inventors measured the enzyme reactivity of magnetic nanoparticles (DNA-binding magnetic nanoparticles) to which each DNA sequence (oligonucleotide probe) prepared in Example 1 was bound.

This will be briefly described as follows:
50 µl of the magnetic nanoparticles to which each oligonucleotide probe was linked reacted with 10 units of nucleases (Endonuclease and Dnase) in 450 µl of a reaction buffer (50 mM Tris HCl (pH 8), 1 mM MgCl₂, 0.1% BSA) for 10 minutes to remove the nanoparticles using a magnet, and then obtained a supernatant containing the DNA probes cleaved by the nucleases. The supernatant reacted with a TMB solution and the chromogenic reaction was observed with a microplate reader (O.D 650 nm).

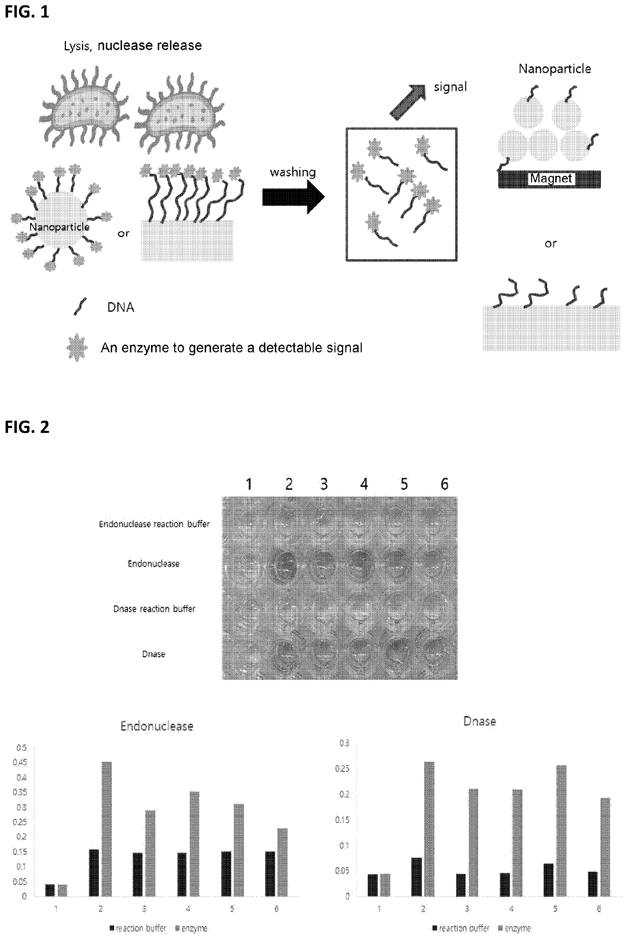

As a result, as illustrated in FIG. 2, it was confirmed that the chromogenic reaction occurred after an enzymatic reaction in all of Experimental Groups 2 to 6.

In addition, even when treated with Dnase, it was also confirmed that all the nanoparticles showed the chromogenic reaction after reaction in a reaction buffer (0.15 M NaCl solution).

Accordingly, it was confirmed that the DNA was well bound to the nanoparticles and that the bound DNA sequence was cleaved by the enzyme to generate a signal.

### Example 3. Microbial culture

*Escherichia coli* and *Acenetobacter baumannii* were inoculated into Luria Bertani (LB) broth and *Staphylococcus aureus* was inoculated into nutrient broth (NB) and cultured in a shaking incubator at 37°C for 14 to 16 hours.

In order to measure colony forming unit (cfu) of the cultured bacteria, 100 µl of the culture was inoculated and smeared on each solid plate medium and then incubated at 37°C for 16 to 24 hours, and the formed colonies were counted and then multiplied with a dilution rate to measure the viable cell count.

In addition, absorbance was measured at a wavelength of 600 nm with a spectrophotometer with respect to an appropriate amount of liquid medium of the cultured bacteria.

### Example 4. Bacterial detection chromogenic (TMB) reaction using magnetic nanoparticle linked to oligonucleotide probe

The present inventors detected bacteria through a chromogenic (TMB) reaction using the probes of the present disclosure.

This will be briefly described as follows:
50 µl of *E. coli* at each concentration reacted with 50 µl of a lysis buffer (stock, 10X, 100 mM KCl, 20 mM Tris (pH 8.5), 5 mM MgCl₂, 1% NP-40, 1% Tween 20 (proteinae K)), and 50 µl of magnetic nanoparticles linked with oligonucleotide probes reacted with 50 µl of a reaction buffer (50 mM Tris-HCl pH 8.0, 5 mM CaCh) to remove the nanoparticles with a magnetic, and signals of DNA probes cleaved by microorganisms were measured. 100 µl of a supernatant reacted with 50 µl of TMB, and the chromogenic reaction was measured using a microplate reader at absorbance of 650 nm.

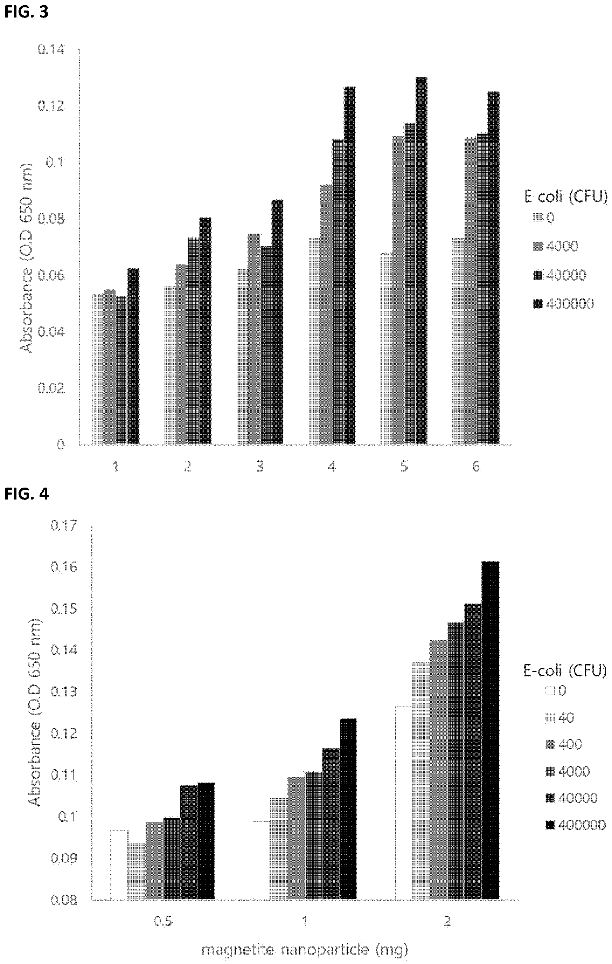

As a result, as illustrated in FIGS. 3 and 4, signals were measured in a concentration-dependent manner in all experimental groups.

In particular, it was confirmed that the signals were further increased in experimental groups 5 and 6 (binding to nanoparticles after DNA annealing).

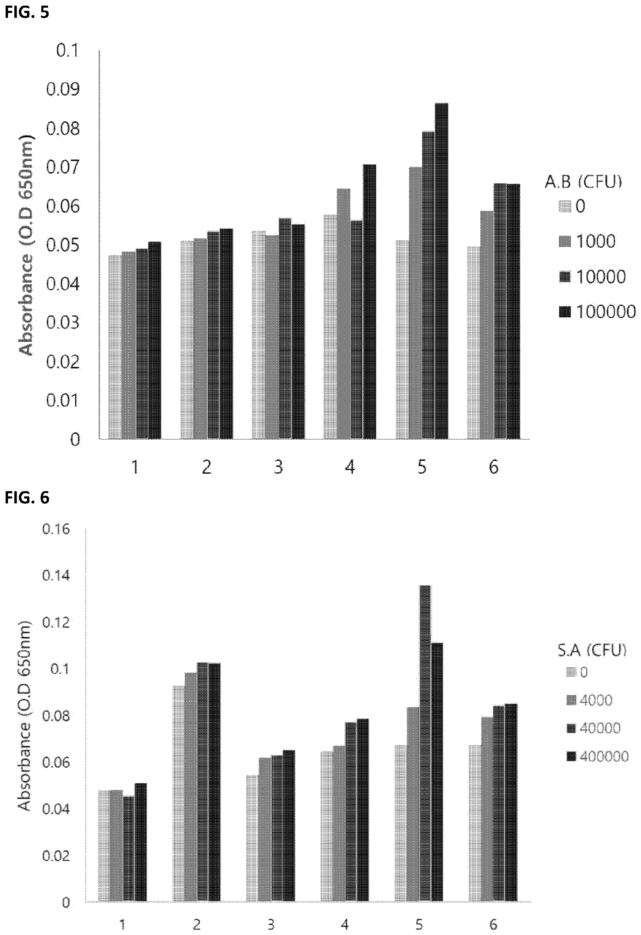

In addition, as illustrated in FIG. 5, even when treated with multidrug-resistant *Acentobacter baumanni,* another gram negative bacteria, signals were measured in a concentration-dependent manner in experimental groups 5 and 6.

In addition, as illustrated in FIG. 6, when treated with *Staphylococcus aureus,* Gram positive bacteria, the most concentration-dependent result was confirmed in No. 6.

As a result, in most of the experimental groups (nanoparticles to which each nucleotide was bound), an increase in signals was confirmed through the reaction with bacteria.

In addition, gold nanoparticles were additionally introduced to bind more enzymes to biotin-DNA-binding magnetic nanoparticles. That is, the signals were increased by binding gold nanoparticle-poly HRP, in which poly-HRP was bound to gold nanoparticles, to biotin-DNA-binding magnetic nanoparticles.

As a result, *E. coli* was able to be detected up to 40 cfu when gold nanoparticle-poly HRP was bound to the nanoparticles as a reaction enzyme.

In addition, the present inventors detected bacteria through a Luminol reaction using the probes of the present disclosure.

This will be briefly described as follows:

50 µl of *E. coli* at each concentration reacted with 50 µl of a lysis buffer (stock, 10, 100 mM KCl, 20 mM Tris (pH 8.5), 5 mM MgCl₂, 1% NP-40, 1% Tween 20 (proteinase K)), and reacted with DNA, 50 µl of magnetic nanoparticles bound to each enzyme (streptavidin-HRP, streptavidin-poly HRP, and gold nanoparticle-streptavidin HRP bound to nanoparticles No. 5, respectively), and 50 µl of a reaction buffer (50 mM Tris-HCl pH 8.0, 5 mM CaCh) to remove nanoparticles with a magnet and measured luminescence reaction signals of the DNA probes cleaved by microorganisms. 100 µl of the supernatant reacted with 100 µl of luminol and 10 µl of H₂O₂ (10 mM), and then a chemiluminescence reaction was measured at a wavelength of 425 nm with a microplate reader.

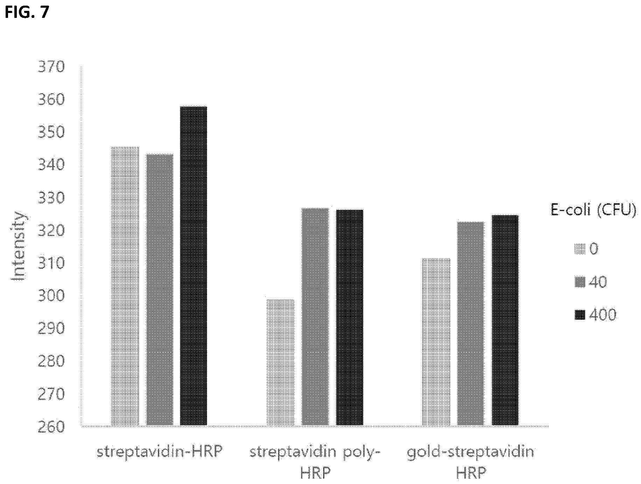

As a result, as illustrated in FIG. 7, a signal higher than that of a control was confirmed at 400 CFU in the case of streptavidin-HRP bound particles, and 40 CFU in the case of polystreptavidin-HRP or gold streptavidin-HRP.

### Example 5. Bacterial detection according to amount of magnetic nanoparticles linked to oligonucleotide probes

The amount of bacteria (*E. coli*) detected according to the amount of magnetic nanoparticles bound was confirmed using nanoparticles No. 5, which had good efficiency. The same amount of gold-poly HRP was bound to each amount of DNA-binding magnetic nanoparticles. 50 µl of *E. coli* at each concentration reacted with 50 µl of a lysis buffer (stock, 10, 100 mM KCl, 20 mM Tris (pH 8.5), 5 mM MgCl₂, 1% NP-40, 1% Tween 20 (proteinase K)), and reacted with 50 µl of magnetic nanoparticles linked with the oligonucleotide probes and 50 µl of a reaction buffer (50 mM Tris-HCl pH 8.0, 5 mM CaCh) to remove nanoparticles with a magnet and measure signals of the DNA probes cleaved by microorganisms. 100 µl of a supernatant reacted with 50 µl of TMB, and the chromogenic reaction was measured using a microplate reader at absorbance of 650 nm.

As a result, as illustrated in FIG. 4, the overall signals increased according to the amount of magnetic nanoparticle binding, and in the measurement values measured after the reaction of *E. coli* at each concentration, a concentration-dependent signal value was able to be confirmed when the amount of nanoparticles was greater than 1 mg.

Overall, according to the present disclosure, it is possible to obtain a detection effect when a nucleic acid probe as a means for linking a solid surface at one end and an enzyme and a luminescent body at the other end is cleaved by a nuclease released during bacterial lysis regardless of a single strand, a double strand, or its length, and even in actual results, it was confirmed that even bacteria of 10² CFU/ml were able to be detected.

Therefore, Gram-negative bacteria or Gram-positive bacteria are able to be quickly and easily detected without false-positive signals by using the microbial contamination detection technology using the magnetic nanoparticles linked to the probes of the present disclosure.

Although the present disclosure has been described in terms of the preferred embodiments described above, various modifications and variations may be made without departing from the spirit and scope of the present disclosure. The appended claims also cover such modifications and variations as fall within the gist of the present disclosure.

## Claims

1. A composition for detecting on-site microbial contamination comprising a solid surface-immobilized oligonucleotide probe.

2. The composition of claim 1, wherein the composition detects contamination of living microorganisms.

3. The composition of claim 2, wherein the microorganisms are Gram-negative bacteria or Gram-positive bacteria.

4. The composition of claim 1, wherein the oligonucleotide probe is immobilized through its 5' or 3'-end on the solid surface.

5. The composition of claim 1, wherein the solid surface is at least one selected from the group consisting of magnetic nanoparticle, well-plate, slide glass, column, porous support, gold, alloy, aluminum, metal oxide, ceramic, quartz, silicon, semiconductor, Si/SiO₂ wafer, germanium, gallium arsenide, carbon, carbon nanotube, polystyrene, polyethylene, polypropylene, polyacrylamide, sepharose, agarose, and colloid.

6. The composition of claim 1, wherein the oligonucleotide probe includes a label generating a detectable signal at its 5'- or 3'-end.

7. The composition of claim 6, wherein the oligonucleotide probe is cleaved by a nucleolytic reaction of the microbial nuclease to release the label from the oligonucleotide probe, resulting in generation of the detectable signal.

8. The composition of claim 7, wherein the label is at least one selected from the group consisting of an enzymatic label, a radioactive label, a fluorescent label, a luminescent label, a chemiluminescent label and a metal label.

9. The composition of claim 1, wherein the solid surface-immobilized oligonucleotide probe further binds to a separate nanoparticle and immobilizes a label on the surface of the separate nanoparticle.

10. The composition of claim 8, wherein the label is linked by biotin.

11. The composition of claim 10, wherein the biotin binds to at least one biotin-affinity protein selected from the group consisting of streptavidin, avidin, traptavidin, and neutravidin.

12. The composition of claim 11, wherein the biotin-affinity protein is labeled by an enzyme.

13. The composition of claim 12, wherein the enzyme is at least one selected from the group consisting of horseradish peroxidase (HRP), alkaline phosphatase (ALP), luciferase, glucokinase, invertase, glucose oxidase, β-D-galactosidase, malate dehydrogenase (MDH) and acetylcholinesterase.

14. The composition of claim 1, wherein the oligonucleotide probe consists of a single strand, a double strand, or a combination thereof.

15. A kit for detecting on-site microbial contamination comprising the composition of any one of claims 1 to 14.

16. A method for detecting on-site microbial contamination comprising the following steps:
(a) dissolving a microorganism in a sample;
(b) treating the sample with the composition of any one of claims 1 to 14; and
(c) detecting a signal released from the sample.

17. The method of claim 16, wherein the detecting of the signal indicates the presence of the microorganism.
